# EUROPEAN PATENT APPLICATION

(11) **EP 4 502 157 A1**
(43) Date of publication of application: **05.02.2025**
(21) Application number: 23780856.3
(22) Date of filing: 30.03.2023
(51) Int. Cl.: C12N 15/09, C12N 1/15, C12N 1/19, C12N 1/21, C12N 5/10, C12N 15/11

(54) **SINGLE-STRAND FORM POLYNUCLEOTIDE AND USE THEREOF IN GENOME EDITING**

(30) Priority: 30.03.2022 JP 2022057755
(71) Applicant: National Institute of Advanced Industrial Science and Technology, Chiyoda-ku Tokyo 100-8921 (JP)
(72) Inventor: MASEDA, Hideaki, Tsukuba-shi Ibaraki 305-8560 (JP)
(74) Representative: Wasner, Marita
(86) International application number: PCT/JP2023/013138
(87) International publication number: WO 2023/190848

(57) **Abstract**

The present invention provides: a single-strand form polynucleotide for editing that is capable of modifying a target site in double-strand genomic DNA and that includes, as a structural unit, a nucleotide analog with a high affinity for DNA; a genome editing kit that includes said polynucleotide for editing; a genome editing kit that includes said polynucleotide for editing and a polynucleotide for promoting editing or an expression vector thereof; a method for modifying a target site in double-strand genomic DNA of a cell or a non-human organism, the method comprising a step for using the genome editing kit to treat a cell or an organism; and a method for producing a cell or an organism in which a target site in double-strand genomic DNA is modified, the method comprising said step.

## Description

### Field

The present invention relates to a single-stranded polynucleotide that can be used for genome editing, and a method for genome editing using the polynucleotide.

### Background

Genome editing technology is a technology for modifying genomic DNA nucleotide sequences by using site-specific nucleases that can recognize and cleave specific nucleotide sequences. In particular, the genome editing technology called the CRISPR/Cas system has attracted attention as an epoch-making method that overcomes the disadvantages of early genome editing methods using ZFN (Zinc-Finger Nuclease) or TALEN (Transcription activator effector-like nuclease), such as difficulty in design, low editing efficiency, and complicated work. The CRISPR/Cas system has been used not only in academic research but also in various fields such as agriculture, forestry, fisheries, livestock breeding, and medical treatment.

The CRISPR/Cas system, however, has a problem of delivery of large site-specific nucleases into cells, as well as a problem of off-target mutations that occur at a different location from the site to be edited, which have been pointed out as issues for practical application.

The present inventor has developed a new genome editing technology using a single-stranded polynucleotide that does not require the delivery of site-specific nucleases (e.g., Patent Literatures 1 and 2). This technology has the advantage that genome editing can be performed by simply expressing or introducing the single-stranded polynucleotide into the cell, without needing to introduce components other than the single-stranded polynucleotide, typically a site-directed nuclease, into the cell.

### Citation List

### Patent Literature

Patent Literature 1: Japanese Patent No. 6899564.
Patent Literature 2: Japanese Patent Application Publication No. 2016-220639.

### Summary

### Problem to be solved

The present invention provides a new means for increasing genome editing efficiency that can be used in genome editing technologies utilizing a single-stranded polynucleotide as described in Patent Literature 1.

### Solution to Problem

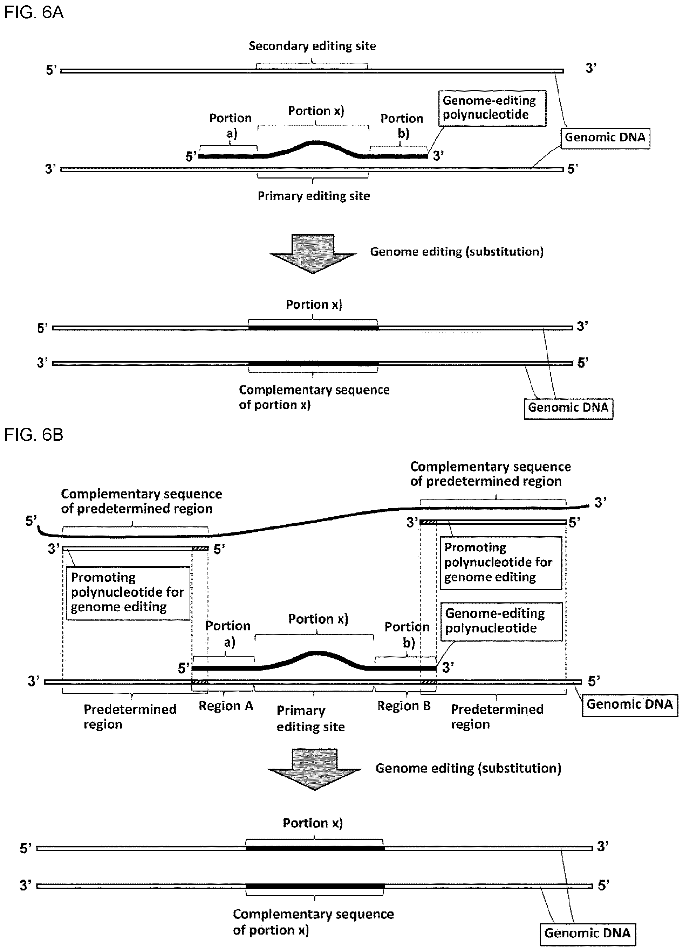

The present inventor has been developing and improving a genome editing method that does not introduce protein components, i.e., a genome editing method using only single-stranded nucleotides, so that the method can be used for actual medical treatment. To increase the editing efficiency, it was essential that the introduced single-stranded nucleotide efficiently binds to the adjacent region of the primary editing site of the genomic DNA (indicated as region A and region B in Fig. 6B). Through several investigations aimed at increasing efficiency, it was found that nucleic acids having the same sequence as the introduced single-stranded nucleotide, i.e., transcripts (RNA) from the region, compete with each other for the adjacent region, which reduces the efficiency of genome editing. The discovery that the efficiency of genome editing can be improved by overcoming the competition led to the present invention. Since there had never been a technical concept that transcripts bind to the aforementioned adjacent regions of the genome and are in a state of competition, and that a method to overcome the competition enhances the efficiency of genome editing by a single-stranded nucleotide, the present invention was made on the basis of this technical concept.

The present inventor has found that the efficiency of genome editing using a single-stranded polynucleotide can be increased by using a single-stranded polynucleotide having a DNA high-affinity nucleotide analogue at a specific position in the nucleotide sequence.

The present disclosure provides the followings.

Item 1A. A single-stranded genome-editing polynucleotide capable of modifying a target site on a double-stranded genomic DNA and containing DNA high-affinity nucleotide analogs,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
   a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b),
at least one of portions a) and b) has a length of 32 nucleotides or more, and contains three or more DNA high-affinity nucleotide analogs within the range of the 30th to 38th nucleotides, one or more DNA high-affinity nucleotide analogs within the range of the 20th to 29th nucleotides, and no DNA high-affinity nucleotide analog within the ranges of the 1st to 2nd nucleotides or the 7th to 16th nucleotides, in the direction from the terminal adjacent to the other portion of the polynucleotide toward the non-adjacent terminal,
the other of the portions a) and b) has a length of 10 nucleotides or more, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

Item 1B. A single-stranded genome-editing polynucleotide capable of modifying a target site on a double-stranded genomic DNA and containing DNA high-affinity nucleotide analogs,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
   a) a portion consisting of a nucleotide sequence having 90% or more identity with the nucleotide sequence of the region adjacent to the 5' terminal side of the secondary editing site,
x) an optional portion consisting of a nucleotide sequence that is not identical to the nucleotide sequence of the secondary editing site, and
b) a portion consisting of a nucleotide sequence having 90% or more identity with the nucleotide sequence of the region adjacent to the 3' terminal side of the secondary editing site, or
the genome-editing polynucleotide consists of, in order from its 5' terminal side, portion a) and portion b),
at least one of portions a) and b) has a length of 32 nucleotides or more, and contains three or more DNA high-affinity nucleotide analogs within the range of the 30th to 38th nucleotides, one or more DNA high-affinity nucleotide analogs within the range of the 20th to 29th nucleotides, and no DNA high-affinity nucleotide analog within the ranges of the 1st to 2nd nucleotides or the 7th to 16th nucleotides, in the direction from the terminal adjacent to the other portion of the polynucleotide toward the non-adjacent terminal,
the other of the portions a) and b) has a length of 10 nucleotides or more, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

Item 1C. A single-stranded genome-editing polynucleotide capable of modifying a target site on a double-stranded genomic DNA and containing DNA high-affinity nucleotide analogs,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
   a) a portion consisting of a nucleotide sequence identical to the nucleotide sequence of the region adjacent to the 5' terminal side of the secondary editing site,
x) an optional portion consisting of a nucleotide sequence that is not identical to the nucleotide sequence of the secondary editing site, and
b) a portion consisting of a nucleotide sequence identical to the nucleotide sequence of the region adjacent to the 3' terminal side of the secondary editing site, or
the genome-editing polynucleotide consists of, in order from its 5' terminal side, portion a) and portion b),
at least one of portions a) and b) has a length of 32 nucleotides or more, and contains three or more DNA high-affinity nucleotide analogs within the range of the 30th to 38th nucleotides, one or more DNA high-affinity nucleotide analogs within the range of the 20th to 29th nucleotides, and no DNA high-affinity nucleotide analog within the ranges of the 1st to 2nd nucleotides or the 7th to 16th nucleotides, in the direction from the terminal adjacent to the other portion of the polynucleotide toward the non-adjacent terminal,
the other of the portions a) and b) has a length of 10 nucleotides or more, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

Item. 2. The genome-editing polynucleotide according to item 1A, 1B or 1C, wherein the lengths of portions a) and b) are each 100 nucleotides or less.

Item. 3. The genome-editing polynucleotide according to item 1A, 1B, 1C or 2, wherein the DNA high-affinity nucleotide analogs are not adjacent to each other.

Item. 4. The genome-editing polynucleotide according to item 1A, 1B, 1C, 2 or 3, wherein the DNA high-affinity nucleotide analogs are nucleotides in which the oxygen atom at the 2' position and the carbon atom at the 4' position of the ribose ring are cross-linked.

Item. 5. A kit for genome editing, containing:
the genome-editing polynucleotide according to item 1A, 1B, 1C, 2, 3 or 4, or
a conjugate of the genome-editing polynucleotide with one or more other substances.

Item 6A. The kit for genome editing according to item 5, further containing:
a promoting polynucleotide for genome editing,
an expression vector thereof, or
a conjugate of the promoting polynucleotide for genome editing or the expression vector thereof with one or more other substances,
wherein the promoting polynucleotide for genome editing is capable of hybridizing to a nucleic acid sequence that is complementary to a predetermined region on the genomic DNA strand on which the primary editing site is set,
the predetermined region overlaps at its 5' terminal part with region A or overlaps at its 3' terminal part with region B on the genomic DNA strand on which the primary editing site is set, in which a region on the genomic DNA strand at a position corresponding to portion a) of the genome-editing polynucleotide is defined as region A, and a region on the genomic DNA strand at a position corresponding to portion b) of the genome-editing polynucleotide is defined as region B, and
the length of overlap with region A at the 5' terminal part of the predetermined region is the same as or shorter than region A, and the length of overlap with region B at the 3' terminal part of the predetermined region is the same as or shorter than region B.

Item 6B. The kit for genome editing according to item 5, further containing:
a promoting polynucleotide for genome editing,
an expression vector thereof, or
a conjugate of the promoting polynucleotide for genome editing or the expression vector thereof with one or more other substances,
wherein the promoting polynucleotide for genome editing consists of a nucleotide sequence having 90% or more identity with the nucleotide sequence of a predetermined region on the genomic DNA strand on which the primary editing site is set,
the predetermined region overlaps at its 5' terminal part with region A or overlaps at its 3' terminal part with region B on the genomic DNA strand on which the primary editing site is set, in which a region on the genomic DNA strand at a position corresponding to portion a) of the genome-editing polynucleotide is defined as region A, and a region on the genomic DNA strand at a position corresponding to portion b) of the genome-editing polynucleotide is defined as region B, and
the length of overlap with region A at the 5' terminal part of the predetermined region is the same as or shorter than region A, and the length of overlap with region B at the 3' terminal part of the predetermined region is the same as or shorter than region B.

Item 6C. The kit for genome editing according to item 5, further containing:
a promoting polynucleotide for genome editing,
an expression vector thereof, or
a conjugate of the promoting polynucleotide for genome editing or the expression vector thereof with one or more other substances,
wherein the promoting polynucleotide for genome editing consists of a nucleotide sequence identical to the nucleotide sequence of a predetermined region on the genomic DNA strand on which the primary editing site is set,
the predetermined region overlaps at its 5' terminal part with region A or overlaps at its 3' terminal part with region B on the genomic DNA strand on which the primary editing site is set, in which a region on the genomic DNA strand at a position corresponding to portion a) of the genome-editing polynucleotide is defined as region A, and a region on the genomic DNA strand at a position corresponding to portion b) of the genome-editing polynucleotide is defined as region B, and
the length of overlap with region A at the 5' terminal part of the predetermined region is the same as or shorter than region A, and the length of overlap with region B at the 3' terminal part of the predetermined region is the same as or shorter than region B.

Item 7. The kit for genome editing according to item 6A, 6B or 6c, wherein the promoting polynucleotide for genome editing has a length of 50 nucleotides or more.

Item 8. The kit for genome editing according to item 6A, 6B, 6c or 7, wherein the overlap has a length of 5 nucleotides or more.

Item 9. The kit for genome editing according to item 6A, 6B, 6c, 7 or 8, containing:
the promoting polynucleotide for genome editing in which the predetermined region is set to overlap at its 5' terminal part with region A,
an expression vector thereof, or
a conjugate of the promoting polynucleotide for genome editing or the expression vector thereof with one or more other substances, and
the promoting polynucleotide for genome editing in which the predetermined region is set to overlap at its 3' terminal part with region B,
an expression vector thereof, or
a conjugate of the promoting polynucleotide for genome editing or the expression vector thereof with one or more other substances.

Item 10. A method for modifying a target site on a double-stranded genomic DNA of a cell or an organism, including the step of treating the cell or the organism using the genome-editing polynucleotide according to item 1A, 1B, 1C, 2, 3 or 4, or the kit for genome editing according to item 6A, 6B, 6c, 7, 8 or 9.

Item 11. A method for producing a cell or an organism in which a target site on a double-stranded genomic DNA has been modified, including the step of treating the cell or the organism using the genome-editing polynucleotide according to item 1A, 1B, 1C, 2, 3 or 4, or the kit for genome editing according to item 6A, 6B, 6c, 7, 8 or 9.

### Advantageous Effects of Invention

According to the present invention, genome editing can be performed efficiently.

### Brief Description of Drawings

[FIG.1] Fig. 1 is a diagram illustrating the relationship between genome-editing polynucleotides having LNA (referred to as LNA-introduced genome-editing polynucleotides) used for genome editing and DNA to be edited. The upper panel illustrates the case of deletion of the central 8 nucleotides and the lower panel illustrates the case of replacement of thymine with adenine near the center.
[FIG.2] Fig. 2 is a diagram illustrating the positions of LNA introduction in LNA-introduced genome-editing polynucleotides used for genome editing of nucleotide deletion.
[FIG.3] Fig. 3 is a graph illustrating the efficiency of genome editing using the LNA-introduced genome-editing polynucleotides.
[FIG.4] Fig. 4 illustrates the positions of LNA introduction (left side of the figure) and genome editing efficiency (right side of the figure) of LNA-introduced genome-editing polynucleotides used for genome editing of nucleotide substitution.
[FIG.5] Fig. 5 is a diagram illustrating the positional relationship between DNA to be edited, a genome-editing polynucleotide, and two types of promoting polynucleotides for genome editing.
[FIG. 6A] Fig. 6A is a diagram illustrating the mechanism of conventional genome editing (substitution) by a genome-editing polynucleotide.
[FIG. 6B] Fig. 6B is a diagram illustrating the mechanism of genome editing (substitution) by a genome-editing polynucleotide and two types of promoting polynucleotides for genome editing.

### Detailed Description of Invention

The following description may be based on representative embodiments or specific examples, but the present invention is not limited to such embodiments or specific examples. The upper limit value and the lower limit value of each numerical range exemplified in the present specification can be combined as desired. In the present specification, a numerical range represented using "to" or"-" means a range including numerical values at both ends thereof as an upper limit value and a lower limit value, unless otherwise noted.

In the present disclosure, genomic DNA refers to DNA contained as a genome in the cell of any living organism. Genomic DNA can be prokaryotic genomic DNA or eukaryotic genomic DNA, which may contain exogenous DNA, such as from a virus, as part of it.

A prokaryote includes, for example, Gram-negative bacteria (Pseudomonas aeruginosa, Escherichia coli, Serratia spp., Sphingomonas spp., Brucella spp., Neisseria gonorrhoeae, Burkholderia spp., Shigella spp., Salmonella spp., Acinetobacter spp., Vibrio cholerae, Klebsiella pneumoniae, Legionella spp., Helicobacter pylori, Campylobacter spp., etc.), and Gram-positive bacteria (Mycobacterium tuberculosis, Mycoplasma spp., Staphylococcus aureus, Actinomyces, Bacillus subtilis, Bacillus anthracis, etc.). A prokaryote can preferably be a Gram-negative bacterium, more preferably Pseudomonas aeruginosa, Escherichia coli, Serratia spp. or Sphingomonas spp, further more preferably Pseudomonas aeruginosa or Escherichia coli.

An example of a eukaryote is an animal, including mammals such as humans and non-human mammals (monkeys, dogs, cats, rabbits, mice, rats, guinea pigs, hamsters, cattle, pigs, goats, sheep, horses, llamas, camels, etc.); birds such as chickens; reptiles such as turtles and crocodiles; amphibians such as African clawed frogs; fish such as zebrafish, medaka, and pufferfish; chordates such as sea squirts; and arthropods such as Drosophila and silkworms.

Another example of a eukaryote is a plant, including food crops such as rice, corn, potatoes, beans, and grains, such as barley and wheat; horticultural crops (vegetables, fruit trees, and flowers), such as leafy vegetables (cabbage, asparagus, etc.), fruit vegetables (eggplant, tomato, cucumber, etc.), root vegetables (radish, carrot, etc.), other vegetables, fruits (e.g., pome fruits such as apples and pears, stone fruits such as Japanese apricots, apricots, plums, peaches, and cherries, nuts such as almonds, walnuts, and chestnuts, and citrus fruits such as tangerines and lemons, and tropical fruit trees such as tropical fruits); and ornamental plants.

Another example of a eukaryote is fungi, including yeast, molds, and basidiomycetes, such as yeast, red bread mold, matsutake mushrooms, shiitake mushrooms, enoki mushrooms, shimeji mushrooms, nameko mushrooms, and eryngii mushrooms. A eukaryote can also be a microalgae.

The sense strand of genomic DNA means to the DNA strand among DNA double strands, that is not the template for transcription within a region that is transcribed into RNA, such as a coding region. The antisense strand means to the complementary strand of the sense strand, which is the template for transcription. The coding region is not limited to a protein-coding region, and also includes a region that codes RNA that does not code a protein (e.g., miRNA, piRNA, tRNA, rRNA, snRNA, gRNA, SRP RNA, pRNA, tncRNA, sbRNA, snIRNA, SLRNA, etc.)

The terms "genome editing", "editing of genomic DNA" and "modification of genomic DNA" can be used interchangeably.

. The term "polynucleotide" refers to a polymer or oligomer formed by multiple deoxyribonucleotides or ribonucleotides linked via phosphodiester bonds and can be used interchangeably with the term "nucleic acid". An example of a polynucleotide includes deoxyribonucleic acid (DNA), ribonucleic acid (RNA), and a chimeric nucleic acid containing both deoxyribonucleotide and ribonucleotide as its constituent units. A polynucleotide can contain a nucleotide analog as its constituent unit.

The terms " portion" and "region" with respect to a polynucleotide can be used interchangeably and both mean a one nucleotide or multiple contiguous nucleotides contained in the polynucleotide. The terms "portion" and "region" with respect to double-stranded genomic DNA can be used interchangeably and both mean a one nucleotide pair or multiple contiguous nucleotide pairs contained in the double-stranded genomic DNA.

In the present disclosure, a portion or region "at a position corresponding to" a portion or region of a polynucleotide refers to a portion or region complementary to the portion or region of that polynucleotide on a different polynucleotide.

Nucleotide sequence identity means to the percentage of identical nucleotides among all overlapping nucleotides in the optimal alignment calculated using algorithms known in the technical field (preferably, the algorithm can consider the gap introduction into one or both sequences for optimal alignment). Identity can be calculated, for example, by aligning two nucleotide sequences using NCBI BLAST (National Center for Biotechnology Information Basic Local Alignment Search Tool) -2 with default settings. When a sequence to be aligned contains uracil, the identity calculation is performed using the sequence where uracil has been replaced with thymine. When a sequence to be aligned contains a modified nucleotide or nucleotide analog, the identity calculation is performed using the sequence where the modified nucleotide or nucleotide analog has been replaced with its corresponding natural deoxyribonucleotide, i.e. adenine, thymine, guanine or cytosine.

In the present disclosure, a polynucleotide or a portion or region thereof that is " capable of hybridizing" to another polynucleotide or a portion or region thereof means that one polynucleotide or a portion or region thereof has the ability to bind to the other polynucleotide or a portion or region thereof through hydrogen bonds between complementary bases. The capability to hybridize can be confirmed by maintaining the hybridized state under a stringent condition. As taught in Berger and Kimmel (1987, Guide to Molecular Cloning Techniques Methods in Enzymology, Vol. 152, Academic Press, San Diego CA), a stringent condition can be determined based on the melting temperature (Tm) of a nucleic acid to which a probe is to be bound. When the hybridization state between two polynucleotides or their portions or regions is maintained by washing under a stringent condition, e.g., with 1 x SSC, 0.1% SDS, at 37°C, they are capable of hybridizing to each other. A stringent condition may be washing with 0.5 x SSC, 0.1% SDS, at 42°C, or with 0.1 x SSC, 0.1% SDS, at 65°C.

The term "expression vector" as used with respect to a polynucleotide refers to a vector containing a polynucleotide and equipped with a mechanism to express the polynucleotide in the cell into which the vector is introduced. A polynucleotide expression vector can also be represented as a vector capable of expressing the polynucleotide in a cell into which the vector is introduced. An example of a polynucleotide expression vector is a vector containing the polynucleotide operably linked to a promoter or other regulatory sequence.

The term "operably linked" as used with respect to a polynucleotide means that a regulatory sequence, such as a promoter, is located sufficiently close to the polynucleotide such that it can influence the expression of the polynucleotide. For example, "a polynucleotide operably linked to a promoter" means that the polynucleotide is linked such that it is expressed under the control of the promoter.

### Genome-editing polynucleotide

The present disclosure provides, in one aspect, a single-stranded genome-editing polynucleotide capable of modifying a target site on a double-stranded genomic DNA and containing DNA high-affinity nucleotide analogs, wherein the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b). The details of the genome-editing polynucleotide are described below (see also Fig. 6A).

In the present disclosure, a target site refers to a site that is intended to be modified on a double-stranded genomic DNA, specifically, one nucleotide pair or multiple contiguous nucleotide pairs, or a position between two contiguous nucleotide pairs on a double-stranded genomic DNA, that is intended to be modified. A target site consists of a primary editing site set on one genomic DNA strand and a secondary editing site on the other genomic DNA strand at a position corresponding to the primary editing site. For example, when the intended modification is to replace or delete one nucleotide pair or multiple contiguous nucleotide pairs in double-stranded genomic DNA, the primary editing site is set at one nucleotide or multiple nucleotides intended to be replaced or deleted on one genomic DNA strand. When the intended modification is to insert one nucleotide pair or multiple contiguous nucleotide pairs into double-stranded genomic DNA, the primary editing site is set between two contiguous nucleotides into which one nucleotide or multiple contiguous nucleotides intended to be inserted on one genomic DNA strand.

The primary editing site can be set at any one nucleotide or multiple contiguous nucleotides, or between any two contiguous nucleotides, on one genomic DNA strand. The primary editing site is not limited to being set at a specific one nucleotide or multiple contiguous nucleotides, or between two specific contiguous nucleotides.

When the primary editing site is set at one nucleotide or multiple contiguous nucleotides, the region adjacent to the 5' terminal side of the primary editing site and the region adjacent to the 3' terminal side of the primary editing site are separated by the length of the primary editing site on the genomic DNA. When the primary editing site is set at a position between two contiguous nucleotides, the region adjacent to the 5' terminal side of the primary editing site and the region adjacent to the 3' terminal side of the primary editing site are adjacent to each other on the genomic DNA.

When the primary editing site is set at multiple contiguous nucleotides on one genomic DNA strand, the number of contiguous nucleotides is not particularly limited, and can be, for example, 2 nucleotides or more, 3 nucleotides or more, 4 nucleotides or more, 5 nucleotides or more, 6 nucleotides or more, 7 nucleotides or more, or 8 nucleotides or more, and can be, for example, 2000 nucleotides or less, 1000 nucleotides or less, 900 nucleotides or less, 800 nucleotides or less, 700 nucleotides or less, 600 nucleotides or less, 500 nucleotides or less, 450 nucleotides or less, 400 nucleotides or less, 350 nucleotides or less, 300 nucleotides or less, 250 nucleotides or less, 200 nucleotides or less, 150 nucleotides or less, 100 nucleotides or less, 90 nucleotides or less, 80 nucleotides or less, 70 nucleotides or less, 60 nucleotides or less, 50 nucleotides or less, 40 nucleotides or less, 30 nucleotides or less, 20 nucleotides or less, 15 nucleotides or less, 12 nucleotides or less, 10 nucleotides or less, or 9 nucleotides or less.

The primary editing site may be set on either strand of the two strands constituting genomic DNA. The secondary editing site is to be set on the strand of genomic DNA on which the primary editing site is not set. For example, when modification of the region of double-stranded genomic DNA that is transcribed into RNA is intended, the primary editing site may be set on the sense strand or on the antisense strand.

Portion a) of the genome-editing polynucleotide can hybridize with the region adjacent to the 3' terminal side of the primary editing site.

Portion a) can also be a portion consisting of a nucleotide sequence having 90% or more identity with the nucleotide sequence of the region adjacent to the 5' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion a) consists of a nucleotide sequence complementary to the nucleotide sequence of the region adjacent to the 3' terminal side of the primary editing site, i.e., a nucleotide sequence identical to the nucleotide sequence of the region adjacent to the 5' terminal side of the secondary editing site.

Portion b) of the genome-editing polynucleotide can hybridize with the region adjacent to the 5' terminal side of the primary editing site.

Portion b) can also be a portion consisting of a nucleotide sequence having 90% or more identity with the nucleotide sequence of the region adjacent to the 3' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion b) consists of a nucleotide sequence complementary to the nucleotide sequence of the region adjacent to the 5' terminal side of the primary editing site, i.e., a nucleotide sequence identical to the nucleotide sequence of the region adjacent to the 3' terminal side of the secondary editing site.

The lengths of portions a) and b) are not limited as long as at least one of them has a length of 32 nucleotides or more, and can be set independently. The length of one of portions a) and b) can be 32 nucleotides or more, for example, 34 nucleotides or more, 35 nucleotides or more, 40 nucleotides or more, 45 nucleotides or more, 50 nucleotides or more, 60 nucleotides or more, 70 nucleotides or more, 80 nucleotides or more, 90 nucleotides or more, or 100 nucleotides or more, and can be 1000 nucleotides or less, 500 nucleotides or less, 300 nucleotides or less, 200 nucleotides or less, 190 nucleotides or less, 180 nucleotides or less, 170 nucleotides or less, 160 nucleotides or less, 150 nucleotides or less, 140 nucleotides or less, 130 nucleotides or less, 120 nucleotides or less, 110 nucleotides or less, 100 nucleotides or less, 90 nucleotides or less, 80 nucleotides or less, 70 nucleotides or less, 60 nucleotides or less, or 50 nucleotides or less; while the length of the other of portions a) and b) can be, for example, 10 nucleotides or more, 20 nucleotides or more, 30 nucleotides or more, 32 nucleotides or more, 34 nucleotides or more, 35 nucleotides or more, 40 nucleotides or more, 45 nucleotides or more, 50 nucleotides or more, 60 nucleotides or more, 70 nucleotides or more, 80 nucleotides or more, 90 nucleotides or more, or 100 nucleotides or more, and can be 1000 nucleotides or less, 500 nucleotides or less, 300 nucleotides or less, 200 nucleotides or less, 190 nucleotides or less, 180 nucleotides or less, 170 nucleotides or less, 160 nucleotides or less, 150 nucleotides or less, 140 nucleotides or less, 130 nucleotides or less, 120 nucleotides or less, 110 nucleotides or less, 100 nucleotides or less, 90 nucleotides or less, 80 nucleotides or less, 70 nucleotides or less, 60 nucleotides or less, or 50 nucleotides or less.

The length of one of portions a) and b) can be, for example, 32 to 1000 nucleotides, 32 to 500 nucleotides, 32 to 300 nucleotides, 32 to 100 nucleotides, 35 to 70 nucleotides, or 35 to 50 nucleotides; while the length of the other of portions a) and b) can be, for example, 10 to 1000 nucleotides, 10 to 500 nucleotides, 10 to 300 nucleotides, 10 to 200 nucleotides, 10 to 100 nucleotides, 32 to 100 nucleotides, 35 to 70 nucleotides, or 35 to 50 nucleotides.

Portion x) is an optional portion consisting of a nucleotide sequence that is not identical to the nucleotide sequence of the secondary editing site and is determined by how the target site is to be modified. For example, when the intended modification is to replace one nucleotide pair or multiple contiguous nucleotide pairs in double-stranded genomic DNA, then portion x) consists of one nucleotide or multiple contiguous nucleotides intended to replace with the secondary editing site. When the intended modification is to delete one nucleotide pair or multiple contiguous nucleotide pairs in double-stranded genomic DNA, then portion x) is not included in the genome-editing polynucleotide. When the intended modification is to insert one nucleotide pair or multiple contiguous nucleotide pairs into double-stranded genomic DNA, then portion x) consists of one nucleotide or multiple contiguous nucleotides intended to be inserted into the secondary editing site.

Portion x) consists of a nucleotide sequence that is not identical to the nucleotide sequence of the secondary editing site. Portion x) can consist of any nucleotide sequence, as long as it is not an exact match to the nucleotide sequence of the secondary editing site, and can consist of, as an example, a nucleotide sequence with less than 90% identity to the nucleotide sequence of the secondary editing site. The identity is, for example, 50% or less, preferably 40% or less, more preferably 30% or less, further more preferably 20% or less, even more preferably 10% or less, and especially preferably 5% or less.

The length of portion x) is not particularly limited, and can be, for example, 2000 nucleotides or less, 1000 nucleotides or less, 900 nucleotides or less, 800 nucleotides or less, 700 nucleotides or less, 600 nucleotides or less, 500 nucleotides or less, 400 nucleotides or less, 300 nucleotides or less, 200 nucleotides or less, 100 nucleotides or less 90 nucleotides or less, 80 nucleotides or less, 70 nucleotides or less, 60 nucleotides or less, 50 nucleotides or less, 40 nucleotides or less, 30 nucleotides or less, 20 nucleotides or less, 10 nucleotides or less, 9 nucleotides or less, 8 nucleotides or less, 7 nucleotides or less, 6 nucleotides or less, 5 nucleotides or less, 4 nucleotides or less, 3 nucleotides or less, 2 nucleotides or less, or 1 nucleotide.

The genome-editing polynucleotide consists of portions a), x) and b), or portions a) and b), in order from the 5' terminal side. Thus, the length of the genome-editing polynucleotide can be determined by summing the lengths of each of the portions consisting of the genome-editing polynucleotide, that is, by summing the lengths of portion a), portion x), and portion b), or by summing the lengths of portions a) and b).

In the genome-editing polynucleotide, at least one of portions a) and b) contains three or more DNA high-affinity nucleotide analogs within the range of the 30th to 38th nucleotides, one or more DNA high-affinity nucleotide analogs within the range of the 20th to 29th nucleotides, and no DNA high-affinity nucleotide analog within the ranges of the 1st to 2nd nucleotides or the 7th to 16th nucleotides, in the direction from the terminal adjacent to the other portion of the polynucleotide toward the non-adjacent terminal.

As an example, in the case of portion a), the terminal adjacent to the other portion refers to the nucleotide adjacent to the 5' terminal nucleotide of portion x) in embodiments where portion x) is included in the genome-editing polynucleotide, and the nucleotide adjacent to the 5' terminal nucleotide of portion b) in embodiments where portion x) is not included in the genome-editing polynucleotide, both of which are the 3' terminal nucleotide of portion a). In addition, the direction toward the non-adjacent terminal to the other portion refers to the direction toward the 5' terminal of portion a). Therefore, the positions of the DNA high-affinity nucleotide analogs in portion a) are defined by counting the 3' terminal nucleotide of portion a) as the first nucleotide toward the 5' terminal direction of portion a).

Similarly, the positions of the DNA high-affinity nucleotide analogs in portion b) are defined by counting the 5' terminal nucleotide of portion b) as the first nucleotide toward the 3' terminal direction of portion b).

The genome-editing polynucleotide contains, in one or both of portions a) and b), three or more DNA high-affinity nucleotide analogs within the range of the 30th to 38th nucleotides, and one or more DNA high-affinity nucleotide analogs within the range of the 20th to 29th nucleotides, in the direction from the terminal adjacent to the other portion of the polynucleotide toward the non-adjacent terminal. The original nucleotide can be replaced with a DNA high-affinity nucleotide analog so that the bases of the original nucleotide and the DNA high-affinity nucleotide analog are common, for example, when the original base is adenine, it can be replaced with a DNA high-affinity nucleotide analog containing adenine or an adenine analog. DNA high-affinity nucleotide analogs can be included at any positions within the ranges of the 30th to 38th nucleotides and the 20th to 29th nucleotides in the direction from the terminal adjacent to the other portion of the polynucleotide toward the non-adjacent terminal. Multiple DNA high-affinity nucleotide analogs may be included adjacently, or they may be included intermittently without being adjacent within the above ranges. When included in both portions a) and b), the positions at which the DNA high-affinity nucleotide analogs are included can be chosen independently, and there's no requirement for the same number of them to be included in both portions.

Portions a) and b) have a length of 32 nucleotides or more when they contain three or more DNA high-affinity nucleotide analogs within the range of the 30th to 38th nucleotides and one or more DNA high-affinity nucleotide analogs within the range of the 20th to 29th nucleotides in the direction from the terminal adjacent to the other portion toward the non-adjacent terminal. Portions a) and b) have a length of 10 nucleotides or more when they are not ones that contain three or more DNA high-affinity nucleotide analogs within the range of the 30th to 38th nucleotides and one or more DNA high-affinity nucleotide analogs within the range of the 20th to 29th nucleotides in the direction from the terminal adjacent to the other portion toward the non-adjacent terminal.

The genome-editing polynucleotide can contain, in one or both of portions a) and b), 3, 4, 5, 6, 7, 8 or 9, preferably 3 to 8 or more, more preferably 3 to 6, even more preferably 3 to 5, and especially preferably 4 to 5 DNA high-affinity nucleotide analogs within the range of the 30th to 38th nucleotides, in the direction from the terminal adjacent to the other portion of the polynucleotide toward the non-adjacent terminal. The genome-editing polynucleotide can contain, in one or both of portions a) and b), 1, 2, 3, 4, 5, 6, 7, 8, 9 or 10, preferably 1 to 6 or more, more preferably 1 to 3, and especially preferably 1 to 2 DNA high-affinity nucleotide analogs within the range of the 20th to 29th nucleotides, in the direction from the terminal adjacent to the other portion of the polynucleotide toward the non-adjacent terminal.

A DNA high-affinity nucleotide analog may further be included at a position not within the ranges of the 30th to 38th nucleotides or the 20th to 29th nucleotides, while it is not included within the ranges of the 1st to 2nd nucleotides or the 7th to 16th nucleotides, in the direction from the terminal adjacent to the other portion of the polynucleotide toward the non-adjacent terminal.

An example of the genome-editing polynucleotide with respect to the positions of the DNA high affinity nucleotide analogs is described below:
a genome-editing polynucleotide that contains three or more DNA high-affinity nucleotide analogs within the range of the 30th to 38th nucleotides, and one or more DNA high-affinity nucleotide analogs within the range of the 20th to 29th nucleotides in the direction from the 3' terminal toward the 5' terminal of portion a),
contains three or more DNA high-affinity nucleotide analogs within the range of the 30th to 38th nucleotides, and one or more DNA high-affinity nucleotide analogs within the range of the 20th to 29th nucleotides in the direction from the 5' terminal toward the 3' terminal of portion b), or
contains three or more DNA high-affinity nucleotide analogs within the range of the 30th to 38th nucleotides and one or more DNA high-affinity nucleotide analogs within the range of the 20th to 29th nucleotides in the direction from the 3' terminal toward the 5' terminal of portion a), and three or more DNA high-affinity nucleotide analogs within the range of the 30th to 38th nucleotides and one or more DNA high-affinity nucleotide analogs within the range of the 20th to 29th nucleotides in the direction from the 5' terminal toward the 3' terminal of portion b); and
contains no DNA high-affinity nucleotide analog within the ranges of the 1st to 2nd nucleotides or the 7th to 16th nucleotides in the direction from the 3' terminal toward the 5' terminal of portion a), or within the ranges of the 1st to 2nd nucleotides or the 7th to 16th nucleotides in the direction from the 5' terminal toward the 3' terminal of portion b).

A preferred example of the genome-editing polynucleotide is a genome-editing polynucleotide that contains 3 to 5 DNA high-affinity nucleotide analogs within the range of the 30th to 38th nucleotides and 1 to 2 DNA high-affinity nucleotide analogs within the range of the 20th to 29th nucleotides in the direction from the 3' terminal toward the 5' terminal of portion a) and 3 to 5 DNA high-affinity nucleotide analogs within the range of the 30th to 38th nucleotides and 1 to 2 DNA high-affinity nucleotide analogs within the range of the 20th to 29th nucleotides in the direction from the 5' terminal toward the 3' terminal of portion b), and contains no DNA high-affinity nucleotide analog within the ranges of the 1st to 2nd nucleotides or the 7th to 16th nucleotides in the direction from the 3' terminal toward the 5' terminal of portion a), or within the ranges of the 1st to 2nd nucleotides or the 7th to 16th nucleotides in the direction from the 5' terminal toward the 3' terminal of portion b).

A DNA high-affinity nucleotide analog is an artificial nucleic acid constituent unit with high binding affinity for DNA that can form polymer structures similar to natural nucleic acids. Examples of a DNA high-affinity nucleotide analog include a nucleotide with a cross-link between the oxygen atom at the 2' position and the carbon atom at the 4' position of the ribose ring, a peptide nucleic acid (PNA) with N-(2-aminoethyl)glycine instead of deoxyribose or ribose, and a morpholino nucleic acid with a morpholine ring instead of deoxyribose or ribose.

In an embodiment, the DNA high-affinity nucleotide analog is a nucleotide in which the oxygen atom at the 2' position and the carbon atom at the 4' position of the ribose ring are cross-linked, examples of which include LNA (Locked Nucleic Acid) in which the oxygen atom at the 2' position and the carbon atom at the 4' position are cross-linked through a methylene bridge, ENA in which they are cross-linked through an ethylene bridge, BNA (Bridged Nucleic Acid) ^{COC} in which they are cross-linked through -CH₂OCH₂-, BNA^{NC} in which they are cross-linked through -NR-CH₂- (R is methyl or a hydrogen atom), cMOE in which they are cross-linked through -CH₂(OCH₃)-, cEt in which they are cross-linked through -CH₂(CH₃)-, AmNA in which they are cross-linked through an amide bridge, and scpBNA in which they are cross-linked through a methylene bridge to form a cyclopropane at the 6' position.

In general, polynucleotides to which DNA high-affinity nucleotide analogs such as PNA and LNA are introduced interact more strongly with their complementary polynucleotides than polynucleotides that do not contain them. However, it was confirmed through experiments by the present inventor that, in polynucleotides used for genome editing, DNA high-affinity nucleotide analogs may rather reduce the efficiency of genome editing depending on their number and positions to be introduced. The present disclosure provides a genome-editing polynucleotide that can be used for genome editing with enhanced efficiency by adjusting the positions and number of DNA high affinity nucleotide analogs introduced into the polynucleotide.

The genome-editing polynucleotide may further contain a chemically modified nucleotide in addition to the DNA high-affinity nucleotide analog as its constituent unit. Examples of a chemically modified nucleotide include a nucleotide in which the phosphate group is replaced with a chemically modified phosphate group such as phosphorothioate (PS), methylphosphonate, and phosphorodithionate, etc., a nucleotide in which the hydroxyl group at the 2' position of the sugar (ribose) portion is replaced with -OR (R represents, for example, CH₃(2'-O-Me), CH₂CH₂OCH₃(2'-MOE), CH₂CH₂NHC(NH)NH₂, CH₂CONHCH₃, CH₂CH₂CN, etc.), a nucleotide in which a methyl group or cationic functional group is introduced at the 5-position of the pyrimidine base, and a nucleotide in which the carbonyl group at the 2-position of the pyrimidine base is replaced with a thiocarbonyl group. Furthermore, examples include a nucleotide in which the phosphate portion or hydroxyl portion is modified with, for example, biotin, an amino group, a lower alkylamine group, an acetyl group, etc., but are not limited to these nucleotides.

The genome-editing polynucleotide containing a DNA high-affinity nucleotide analog and optionally a chemically modified nucleotide can be produced by known synthetic methods.

The genome-editing polynucleotide in the present disclosure can modify genomic DNA in a cell as desired, similar to the single-stranded polynucleotide disclosed in Patent Literature 1 (Japanese Patent No. 6899564). In the modification, the genome-editing polynucleotide does not require the introduction of site-specific nucleases (ZFN proteins, TALEN proteins, Cas proteins, etc.), guide RNAs, or their expression vectors into the cell, as is the case with conventional genome-editing technologies.

Although not bound by theory, portion a) of the genome-editing polynucleotide hybridizes with the region adjacent to the 3' terminal side of the primary editing site set on one genomic DNA strand, while portion b) hybridizes with the region adjacent to the 5' terminal side of the primary editing site. As a result, when the genome-editing polynucleotide consists of portions a), x), and b), the portion x) protrudes, and when the genome-editing polynucleotide consists of portions a) and b), the primary editing site on the genomic DNA protrudes, without hybridizing to other sequences. This protruding segment is recognized by the repair system, allowing for modification of the target site on the double-stranded genomic DNA.

Specifically, when it is desired to insert one nucleotide pair or multiple contiguous nucleotide pairs in double-stranded genomic DNA, the primary editing site is set between two contiguous nucleotides where insertion is desired on one genomic DNA strand; portions a) and b) are set as described above; and portion x) is set at one nucleotide or multiple contiguous nucleotides that are desired to be inserted into the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site.

If it is desired to replace one nucleotide pair or multiple contiguous nucleotide pairs in double-stranded genomic DNA, the primary editing site is set at one nucleotide or multiple contiguous nucleotides where substitution is desired on one genomic DNA strand; portions a) and b) are set as described above; and portion x) is set at one nucleotide or multiple contiguous nucleotides that are desired to be replaced with the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site (shown in Fig. 6A).

If it is desired to delete one nucleotide pair or multiple contiguous nucleotide pairs in double-stranded genomic DNA, the primary editing site is set at one nucleotide or multiple contiguous nucleotides where deletion is desired on one genomic DNA strand; and portions a) and b) are set as described above. By performing genome editing using the genome-editing polynucleotide consisting of portions a) and b) designed in this manner, the primary editing site can be deleted from one genomic DNA strand and the secondary editing site can be deleted from the other genomic DNA strand, i.e., the target site can be deleted from the double-stranded genomic DNA.

Although not bound by theory, considering that there are many transcribed RNAs in the vicinity of genomic DNA, for example, when the target site is in a genomic DNA region that is transcribed into RNA and the primary editing site is set on the genomic DNA sense strand, the genome-editing efficiency is suggested to be not good. This is because the genome-editing polynucleotide may bind, rather than to the region adjacent to the 3' terminal side of the primary editing site and the region adjacent to the 5' terminal side of the primary editing site on the genomic DNA sense strand, preferentially to the region with the same nucleotide sequence as the above two regions in the surrounding RNAs. When genome-editing efficiency is a priority, it is preferable to set the primary editing site on the antisense strand rather than on the sense strand of genomic DNA.

The genome-editing polynucleotide in the present disclosure contains DNA high-affinity nucleotide analogs at the specific positions as described above, thereby enabling it to edit genomic DNA more efficiently than a genome-editing polynucleotide without DNA high-affinity nucleotide analogs.

As described above, portion a) of the genome-editing polynucleotide can hybridize with the region adjacent to the 3' terminal side of the primary editing site set on one genomic DNA strand, and portion b) of the genome-editing polynucleotide can hybridize with the region adjacent to the 5' terminal side of the primary editing site, thereby modifying the target site on the double-stranded genomic DNA. Therefore, the genome-editing polynucleotide may have any nucleotide sequence added to one or both ends, i.e., to the 5' terminal of portion a) and/or to the 3' terminal of portion b), as long as the addition of the sequence does not affect hybridization of portion a) to the region adjacent to the 3' terminal side of the primary editing site, and portion b) to the 5' terminal side of the primary editing site. The genome-editing polynucleotide in the present disclosure encompasses those to which such an optional nucleotide sequence is added.

For the genome-editing polynucleotide with an optional nucleotide sequence added at its terminal, calculation of the identity with each of the regions adjacent to the 5' terminal side and the 3' terminal side of the secondary editing site is performed between the nucleotide sequence of portion a) and the nucleotide sequence of the region adjacent to the 5' terminal side of the secondary editing site, and between the nucleotide sequence of portion b) and the nucleotide sequence of the region adjacent to the 3' terminal side of the secondary editing site. The added optional nucleotide sequence is considered to be absent in the identity calculation.

### Promoting polynucleotide for genome editing

The genome-editing polynucleotide can be used in combination with a single-stranded promoting polynucleotide for genome editing to improve the genome-editing efficiency. The promoting polynucleotide for genome editing is a polynucleotide that is capable of hybridizing to a nucleic acid sequence that is complementary to a predetermined region (also referred to as a complementary sequence of the predetermined region) on a genomic DNA strand on which the primary editing site is set. The details of the promoting polynucleotide for genome editing are described below (see also Fig. 6B).

The predetermined region is set to overlap at its 5' terminal part with region A or at its 3' terminal part with region B. On the genomic DNA strand where the primary editing site is set, a region at a position corresponding to portion a) of the genome-editing polynucleotide is defined as region A, and a region at a position corresponding to portion b) of the genome-editing polynucleotide is defined as region B.

The length of overlap at the 5' terminal part of the predetermined region with region A is not limited as long as it is the same as or shorter than that of region A. The length of overlap at the 3' terminal part of the predetermined region with region B is not limited as long as it is the same as or shorter than that of region B. The length of these overlaps can be set independently, and can be, for example, 1 nucleotide or more, 2 nucleotides or more, 3 nucleotides or more, 4 nucleotides or more, 5 nucleotides or more, 6 nucleotides or more, 7 nucleotides or more, 8 nucleotides or more, 9 nucleotides or more, or 10 nucleotides or more, and can be 20 nucleotides or less, 19 nucleotides or less, 18 nucleotides or less, 17 nucleotides or less, 16 nucleotides or less, 15 nucleotides or less, 14 nucleotides or less, 13 nucleotides or less, 12 nucleotides or less, or 11 nucleotides or less. The length of these overlaps can also be independently, for example, 1 to 20 nucleotides, 5 to 20 nucleotides, 5 to 15 nucleotides, 8 to 12 nucleotides, or 9 to 11 nucleotides.

The complementary sequence of the predetermined region is a region on another polynucleotide that is complementary to the predetermined region on the genomic DNA strand. The complementary sequence of the predetermined region is typically found in nature in RNAs transcribed from the genomic DNA strand on which the predetermined region is set.

The promoting polynucleotide for genome editing can hybridize to the complementary sequence of the predetermined region on the genomic DNA strand on which the primary editing site is set.

The promoting polynucleotide for genome editing may be a polynucleotide consisting of a nucleotide sequence having 90% or more identity with the nucleotide sequence of the predetermined region. Here, the identity is preferably 95% or more, more preferably 97% or more, further more preferably 99% or more, even more preferably 99.5% or more, and especially preferably 99.9% or more.

In a preferred embodiment, the promoting polynucleotide for genome editing consists of a nucleotide sequence identical to the nucleotide sequence of the predetermined region described above.

The promoting polynucleotide for genome editing includes two types of promoting polynucleotides: a promoting polynucleotide for genome editing in which the predetermined region is set to overlap at its 5' terminal part with region A, and a promoting polynucleotide for genome editing in which the predetermined region is set to overlap at its 3' terminal part with region B. The genome-editing efficiency can be improved by using one of these two types of promoting polynucleotides for genome editing in combination with the genome-editing polynucleotide. The genome-editing efficiency can be further improved by using both of these two types of promoting polynucleotides for genome editing in combination with the genome-editing polynucleotide.

In an embodiment using the above two types of promoting polynucleotides for genome editing, the ratio of the two types of promoting polynucleotides for genome editing used is not limited, and the molar concentrations of the two may be comparable, or the molar concentration of one may be much higher than that of the other.

The length of the promoting polynucleotide for genome editing is the same as or longer than the length of the overlap with region A, when the predetermined region is set to overlap at its 5' terminal part with region A. The length of the promoting polynucleotide for genome editing is the same as or longer than the length of the overlap with region B, when the predetermined region is set to overlap at its 3' terminal part with region B. When the two types of promoting polynucleotides for genome editing are used, the length of each promoting polynucleotide for genome editing does not have to be the same and can be set independently.

The length of the promoting polynucleotide for genome editing can independently be, for example, 20 nucleotides or more, 30 nucleotides or more, 40 nucleotides or more, 50 nucleotides or more, 60 nucleotides or more, 70 nucleotides or more, 80 nucleotides or more, 90 nucleotides or more, 100 nucleotides or more, 110 nucleotides or more, 120 nucleotides or more, or 130 nucleotides or more, and can be 1000 nucleotides or less, 500 nucleotides or less, 300 nucleotides or less, 250 nucleotides or less, 200 nucleotides or less, 190 nucleotides or less, 180 nucleotides or less, 170 nucleotides or less, 160 nucleotides or less, 150 nucleotides or less, 140 nucleotides or less or 130 nucleotides or less.

The length of the promoting polynucleotide for genome editing can independently be, for example, 10 to 1000 nucleotides, 20 to 500 nucleotides, 30 to 300 nucleotides, 40 to 200 nucleotides, 50 to 200 nucleotides, 60 to 200 nucleotides, 70 to 150 nucleotides, or 70 to 130 nucleotides.

Although not bound by theory, in an environment where there are many other polynucleotides that have regions complementary to the primary editing site or its neighboring regions (namely region A and region B described above), the binding between the genome-editing polynucleotide and the genomic DNA strand on which the primary editing site is set may be competitively inhibited by these other polynucleotides. Consequently, the genome-editing efficiency by the genome-editing polynucleotide is thought to be reduced.

For example, when the target site is located in a genomic DNA region that is transcribed into RNA and the primary editing site is set on the genomic DNA antisense strand, the genome-editing polynucleotide may compete with the surrounding RNA for binding to the genomic DNA antisense strand. This competition may inhibit the binding between the genome-editing polynucleotide and the genomic DNA antisense strand. Consequently, the genome-editing efficiency is thought to be lower than in cases where no surrounding RNA is present.

In such an environment, when the genome-editing polynucleotide coexists with the promoting polynucleotide for genome editing, the aforementioned other polynucleotides (e.g., RNA) can bind to the promoting polynucleotide for genome editing. Consequently, the inhibition of binding between the genome-editing polynucleotide and the genomic DNA strand (e.g., antisense strand) on which the primary editing site is set can be alleviated, and the genome-editing efficiency is expected to improve (Fig. 6B).

In an embodiment in which the target site is located in a genomic DNA region that is transcribed into RNA and the primary editing site is set on the genomic DNA antisense strand, it is preferable for the promoting polynucleotide for genome editing to have a high binding affinity for the RNA. The promoting polynucleotide for genome editing may contain one or more RNA high-affinity nucleotide analogs. The original nucleotide can be replaced with an RNA high-affinity nucleotide analog so that the bases of the original nucleotide and the RNA high-affinity nucleotide analog are common, for example, when the original base is adenine, it can be replaced with an RNA high-affinity nucleotide analog containing adenine or an adenine analog. Multiple RNA high-affinity nucleotide analogs may be included adjacently, or they may be included intermittently without being adjacent. When the two types of promoting polynucleotides for genome editing are used, each promoting polynucleotide for genome editing can contain an RNA high affinity nucleotide analog at a position selected independently of each other. The number of the nucleotide analogs does not have to be the same.

Although not bound by theory, in an embodiment in which the target site is located in a genomic DNA region that is transcribed into RNA and the primary editing site is set on the genomic DNA antisense strand, when the target site is set in a region that is transcribed into an intron part of mRNA precursor, there would be more intense competition between the genome-editing polynucleotide and RNA for binding to the genomic DNA antisense strand, and the genome-editing efficiency is thought to be lower compared to when the target site is set in a region that is transcribed into an exon part of mRNA precursor. This is because a large number of intron parts, excised by splicing from the mRNA precursor, are present in the vicinity of the genomic DNA.

Therefore, the promoting polynucleotide for genome editing can improve the genome-editing efficiency both when the target site is located in a genomic DNA region that is transcribed into an intron part of mRNA precursor and the primary editing site is set on the genomic DNA antisense strand, and when the target site is located in a genomic DNA region that is transcribed into an exon part of mRNA precursor and the primary editing site is set on the genomic DNA antisense strand. In particular, when the target site is located in a genomic DNA region that is transcribed into an intron part of mRNA precursor and the primary editing site is set on the genomic DNA antisense strand, the promoting polynucleotide for genome editing can exhibit a strong effect on improvement in genome-editing efficiency.

### Kit for genome editing

The present disclosure provides, in one aspect, a kit for genome editing that contains the aforementioned genome-editing polynucleotide.

The present disclosure provides, in one aspect, a kit for genome editing that contains the aforementioned genome-editing polynucleotide and the aforementioned promoting polynucleotide for genome editing. The kit for genome editing preferably contains two types of the promoting polynucleotides for genome editing: the promoting polynucleotide for genome editing in which the predetermined region is set to overlap at its 5' terminal part with region A, and the promoting polynucleotide for genome editing in which the predetermined region is set to overlap at its 3' terminal part with region B.

The kit for genome editing can contain, instead of the genome-editing polynucleotide, a conjugate of the genome-editing polynucleotide with one or more other substances. The kit for genome editing can contain, instead of the promoting polynucleotide for genome editing, an expression vector of the promoting polynucleotide for genome editing, or a conjugate of the promoting polynucleotide for genome editing or the expression vector of the promoting polynucleotide for genome editing with one or more other substances.

Other substances conjugated to the genome-editing polynucleotide are not limited as long as they can form a conjugate with the genome-editing polynucleotide and do not interfere with the function of the genome-editing polynucleotide, i.e., the function of modifying the target site on genomic DNA. Other substances conjugated to the promoting polynucleotide for genome editing are not limited as long as they can form a conjugate with the promoting polynucleotide for genome editing and do not interfere with the function of the promoting polynucleotide for genome editing, i.e., the function of improving genome-editing efficiency by the genome-editing polynucleotide.

Examples of other substances conjugated to the genome-editing polynucleotide and the promoting polynucleotide for genome editing include functional peptides such as nuclear transfer signals and membrane-permeable peptides; hydrophilic polymers such as polyethylene glycol and dextran; and labeling substances such as radioisotopes, fluorescein and its derivatives, rhodamine and its derivatives, fluorescent proteins, phycobiliproteins, biotin, and avidin.

The kit for genome editing can further contain a reagent including a nucleic acid transfer reagent such as a transfection reagent, and a buffer solution.

The components of the kit for genome editing, such as the aforementioned genome-editing polynucleotide or its conjugate, the aforementioned promoting polynucleotide for genome editing, its expression vector or its conjugate, each of the reagents, may be contained in the kit for genome editing in separate forms, or may be contained in the kit for genome editing in the form of a composition in which multiple components are combined.

The kit for genome editing may further contain, for example, an instrument to be used in genome editing, instructions for use.

In an embodiment, the kit for genome editing is a kit for genome editing for the treatment or prevention of a disease, and can contain, in addition to a therapeutically or prophylactically effective amount of the genome-editing polynucleotide or a conjugate of the genome-editing polynucleotide with one or more other substances, other pharmaceutically acceptable components, such as a pharmaceutically acceptable additive (buffer, stabilizer, preservative, excipient, etc.), and a pharmaceutically acceptable medium (water, saline, phosphate buffered saline (PBS), etc.). The kit for genome editing for the treatment or prevention of a disease can be used to treat or prevent a disease caused by an abnormality in genomic DNA.

### Method for genome editing and method for producing genome-edited cell or organism

The present disclosure provides, in one aspect, a method for modifying a target site on double-stranded genomic DNA of a cell or an organism (also referred to as a "method for genome editing" in the present disclosure), including the step of treating the cell or the organism using the aforementioned genome-editing polynucleotide or the aforementioned kit for genome editing. The present disclosure also provides, in one aspect, a method for producing a cell or an organism in which a target site on double-stranded genomic DNA has been modified (also referred to as a "method for producing a genome-edited cell or organism" in the present disclosure), including the step of treating the cell or the organism using the aforementioned genome-editing polynucleotide or the aforementioned kit for genome editing.

The cell to be treated using the genome-editing polynucleotide or the kit for genome editing is a cell whose genomic DNA is desired to be modified and can originate from a variety of organisms, including the prokaryotes and eukaryotes mentioned above. If the cell is a cell of a multicellular organism, the cell can be derived from various tissues of the organism. The cell may be a somatic cell or a germ cell. The cell may be a differentiated or undifferentiated cell, a tissue stem cell, an embryonic stem cell, or an induced pluripotent stem cell (iPS cell).

The cell to be treated using the genome-editing polynucleotide or the kit for genome editing may be in the form of a single cell or a cell population containing one or more types of cells.

The organism to be treated using the genome-editing polynucleotide or the kit for genome editing is an organism whose genomic DNA is desired to be modified and can be a variety of organisms, including the prokaryotes and eukaryotes mentioned above. The organism may be a mature individual, an embryo or fetus in the developmental stage, or in the case of plants, a seed, seedling, or bulb.

In an embodiment, the cell can be represented as a cell excluding human gametes and fertilized eggs.

In an embodiment, the organism excludes humans and can be represented as a non-human organism. In another embodiment, the organism excludes humans and animals and can be represented as a non-human, non-animal organism.

Further, in an embodiment, the cell excludes human gametes and fertilized eggs, and the organism excludes humans. In another preferred embodiment, the cell excludes human gametes and fertilized eggs, and the organism excludes humans and animals.

Treatment of the cell or organism using the genome-editing polynucleotide or the kit for genome editing means making the genome-editing polynucleotide or a conjugate of the genome-editing polynucleotide with one or more other substances, or making the genome-editing polynucleotide or a conjugate of the genome-editing polynucleotide with one or more other substances and the promoting polynucleotide for genome editing, its expression vector, or a conjugate of the promoting polynucleotide for genome editing or its expression vector with one or more other substances contained in the kit for genome editing, coexist with the cell, or administering them to the organism. By this coexistence with the cell or administration to the organism, the genome-editing polynucleotide or a conjugate of the genome-editing polynucleotide with one or more other substances are, or the genome-editing polynucleotide or a conjugate of the genome-editing polynucleotide with one or more other substances and the promoting polynucleotide for genome editing, its expression vector, or a conjugate of the promoting polynucleotide for genome editing or its expression vector with one or more other substances are introduced into the cell or organism, thereby the genomic DNA in the cell or organism can be edited.

Treatment of the cell or organism using the genome-editing polynucleotide or the kit for genome editing can be performed by various known methods selected by those skilled in the art in consideration of factors such as the type or nature of the cell or organism. Treatment of the cell or organism can include, for example, microinjection, electroporation, DEAE-dextran treatment, transfection using a transfection reagent (jetPEI (PolyPlus-transfection), Lipofectamine, etc.), transfection using a lipid nanoparticle, and the hydrodynamic method.

In an embodiment, the treatment of a human cell is performed in vitro or ex vivo.

In general, genome editing may increase or decrease the characteristic of a cell or organism, such as nutrient requirements, sensitivity or resistance to chemicals, may confer a characteristic not present in the cell or organism before editing, or may cause the cell or organism to lose a characteristic it had before editing. The desired genome-edited cell or organism can be selected using such a change in characteristic as an indicator. For example, if genome editing is expected to increase or confer drug resistance, the genome-edited cell or organism can be selected by culturing cells treated using the genome-editing polynucleotide or the kit for genome editing with an appropriate medium containing the drug, or raising organisms treated using the genome-editing polynucleotide or the kit for genome editing with an appropriate feed containing the drug. The method of selection and evaluation of the characteristic of the cells or organisms, and the culture or rearing conditions that enable selection of the cells or organisms can be determined as appropriate within the ordinary practicing capability of those skilled in the art.

The method for genome editing and the method for producing a genome-edited cell or organism described above can include, in addition to the step of treating the cell or organism using the genome-editing polynucleotide or the kit for genome editing, the step of selecting a cell or organism whose genomic DNA has been edited using a characteristic of the cell or organism that is changed by the genome-editing polynucleotide as an indicator.

While the invention will be described in more detail by the following examples, it should not be considered as being limited to these examples.

### [Examples]

### LNA-introduced genome-editing polynucleotide

Single-stranded DNAs consisting of the nucleotide sequences shown in SEQ ID Nos: 1-8 were synthesized by MacroGen or Eurofin and used as genome-editing polynucleotides without LNA. Twenty-eight single-stranded DNAs (SEQ ID NOs: 9-36), in which LNAs were introduced as part of the nucleotide sequences shown in SEQ ID NOs: 1-8, were synthesized from Gene Design Inc. on consignment and used as LNA-introduced genome-editing polynucleotides. Tables 1 and 2 show the nucleotide sequence, the positions of the introduced LNAs, and the distances of the LNAs from the deletion or substitution sites for each genome-editing polynucleotide. In the nucleotide sequences in the tables, nucleotides (a, t, g, c) in lower case letters are natural-type nucleotides (adenine, thymine, guanine, cytosine), A (L) is adenine of LNA type, T (L) is thymine of LNA type, G (L) is guanine of LNA type, 5 (L) is 5-methylcytosine of LNA type.

**[Table 1]**

| Name | Length (mer) | nucleotide sequence (5'→3') | SEQ ID NO: | Position of LNA from 5' terminal | When used for deletion (Example 1) | | When used for substitution (Example 2) | |
|---|---|---|---|---|---|---|---|---|
| | | | | | Distance (number of nucleotides) of LNA(s) present in portion a) from 3' terminal of portion a) | Distance (number of nucleotides) of LNA(s) present in portion b) from 5' terminal of portion b) | Distance (number of nucleotides) of LNA(s) present in portion a) from 3' terminal of portion a) | Distance (number of nucleotides) of LNA(s) present in portion b) from 5' terminal of portion b) |
| DNA-20 | 20 | cccgaccacatgaagcagca | 1 | | | | | |
| DNA-40 | 40 | cagccgctaccccgaccacatgaagcagcacgacttcttc | 2 | | | | | |
| DNA-60 | 60 | tgcagtgcttcagccgctaccccgaccacatgaagcagcacgacttcttcaagtccgcca | 3 | | | | | |
| DNA-70 | 70 | cggcgtgcagtgcttcagccgctaccccgaccacatgaagcagcacgacttcttcaagtccgcca tgccc | 4 | | | | | |
| DNA-80 | 80 | acctacggcgtgcagtgcttcagccgctaccccgaccacatgaagcagcacgacttcttcaagtc cgccatgcccgaagg | 5 | | | | | |
| DNA-90 | 90 | ccctgacctacggcgtgcagtgcttcagccgctaccccgaccacatgaagcagcacgacttcttc aagtccgccatgcccgaaggctacg | 6 | | | | | |
| DNA-100 | 100 | gaccaccctgacctacggcgtgcagtgcttcagccgctaccccgaccacatgaagcagcacga cttcttcaagtccgccatgcccgaaggctacgtccag | 7 | | | | | |
| DNA-120 | 120 | ccaccctcgtgaccaccctgacctacggcgtgcagtgcttcagccgctaccccgaccacatgaa gcagcacgacttcttcaagtccgccatgcccgaaggctacgtccaggagcgcacca | 8 | | | | | |
| 20-10L | 20 | 5(L)c5(L)gA(L)c5(L)a5(L)atG(L)aA(L)g5(L)aG(L)cA(L) | 9 | 1, 3, 5, 7, 9, 12, 14, 16, 18, 20 | 10, 8, 6, 4, 2 | 2, 4, 6, 8, 10 | | |
| 40-10L | 40 | 5(L)aG(L)c5(L)g5(L)tA(L)ccccgaccacatgaagcagcacG(L)a5(L)tT(L)cT(L)t 5(L) | 10 | 1, 3, 5, 7, 9, 32, 34, 36, 38, 40 | 20, 18, 16, 14, 12 | 12, 14, 16, 18, 20 | | |
| 60-10L | 60 | T(L)g5(L)aG(L)tG(L)cT(L)tcagccgctaccccgaccacatgaagcagcacgacttcttca A(L)gT(L)c5(L)g5(L)cA(L) | 11 | 1, 3, 5, 7, 9, 52, 54, 56, 58, 60 | 30, 28, 26, 24, 22 | 22, 24, 26, 28, 30 | | |
| 60-12L | 60 | T(L)g5(L)aG(L)tG(L)cT(L)t5(L)agccgctaccccgaccacatgaagcagcacgacttct t5(L)aA(L)gT(L)c5(L)g5(L)cA(L) | 12 | 1, 3, 5, 7, 9, 11, 50, 52, 54, 56, 58, 60 | 30, 28, 26, 24, 22, 20 | 20, 22, 24, 26, 28, 30, 32 | | |
| 70-(10-2)L | 70 | cgG(L)cG(L)tG(L)cA(L)gtgcttcagccgctaccccgaccacatgaagcagcacgacttct tcaagtccG(L)c5(L)aT(L)g5(L)cc | 13 | 3, 5, 7, 9, 62, 64, 66, 66 | 33, 31, 29, 27 | 27, 29, 31, 33 | | |
| 70-(10-2)L-40c2L | 70 | cgG(L)cG(L)tG(L)cA(L)gtgctT(L)cagccgctaccccgaccacatgaagcagcacgac ttcttcA(L)agtccG(L)c5(L)aT(L)g5(L)cc | 14 | 3, 5 7, 9 15, 56, 62, 64, 66, 68 | 33, 31, 29, 27, 21 | 21, 27, 29, 31, 33 | | |
| 70-(10-4)L | 70 | cggcG(L)tG(L)cA(L)gtgcttcagccgctaccccgaccacatgaagcagcacgacttcttca agtccG(L)cc5(L)aT(L)gccc | 15 | 5, 7, 9, 62, 64, 66 | 31. 29, 27 | 27, 29, 31 | | |
| 70-(10-6)L | 70 | cggcgtG(L)cA(L)gtgcttcagccgctaccccgaccacatgaagcagcacgacttcttcaagt ccG(L)cc5(L)atgccc | 16 | 7, 9, 62, 64 | 29, 27 | 27, 29 | | |
| 70-(10-8)L | 70 | cggcgtgcA(L)gtgcttcagccgctaccccgaccacatgaagcagcacgacttcttcaagtccG (L)ccatgccc | 17 | 9, 62 | 27 | 27 | | |
| 70-10L | 70 | 5(L)gG(L)cG(L)tG(L)cA(L)gtgcttcagccgctaccccgaccacatgaagcagcacgact tcttcaagtccG(L)c5(L)aT(L)g5(L)c5(L) | 18 | 1, 3, 5, 7, 9, 62, 64, 66, 68, 70 | 35, 33, 31, 29, 27 | 27, 29, 31, 33, 35 | 37, 35, 33, 31, 29 | 24, 26, 28, 30, 32 |
| 70-10L-40c2L | 70 | 5(L)gG(L)cG(L)tG(L)cA(L)gtgctT(L)cagccgctaccccgaccacatgaagcagcac gacttcttcA(L)agtccG(L)c5(L)aT(L)g5(L)c5(L) | 19 | 1, 3, 5, 7, 9, 15, 56, 62, 64, 66, 68, 70 | 35, 33, 31, 29, 27, 21 | 21, 27, 29, 31, 33, 35 | 37, 35, 33, 31, 29, 23 | 18,24,26 28, 30, 32 |
| 80-10L | 80 | A(L)c5(L)tA(L)cG(L)g5(L)gtgcagtgcttcagccgctaccccgaccacatgaagcagca cgacttcttcaagtccgccatG(L)c5(L)cG(L)aA(L)gG(L) | 20 | 1, 3, 5, 7, 9, 72, 74, 76, 78, 80 | 40, 38, 36, 34, 32 | 32, 34, 36, 38, 40 | | |

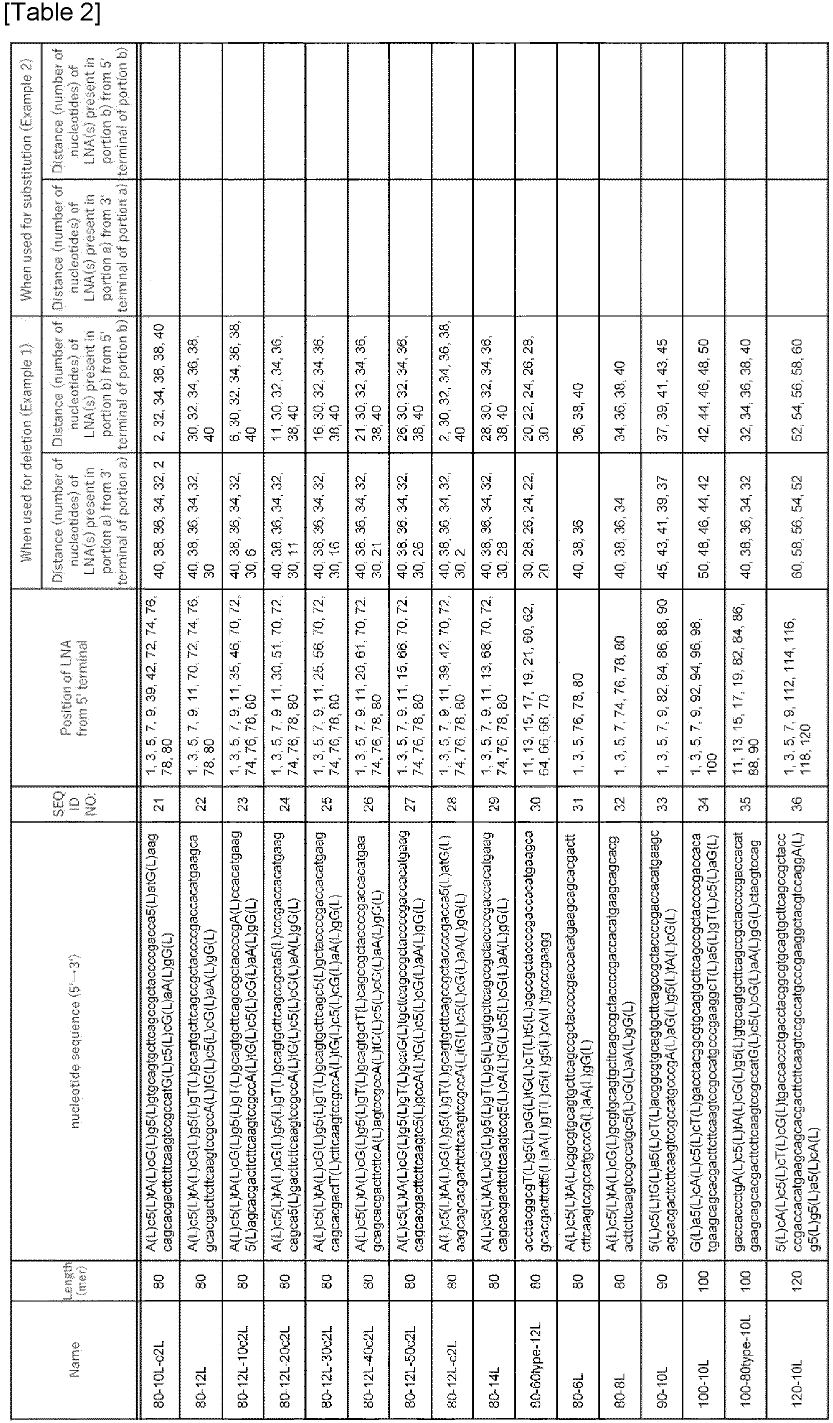

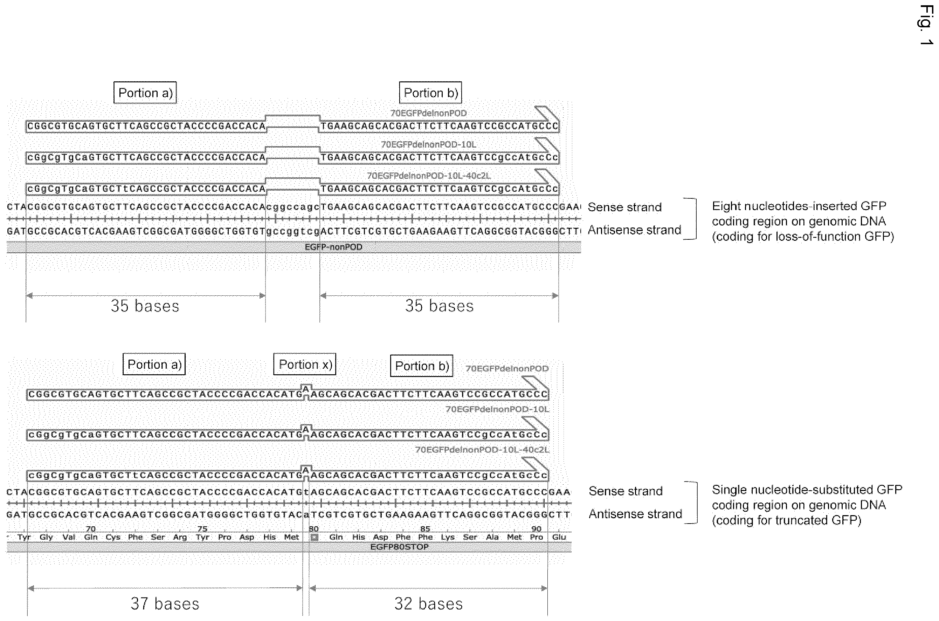

The single-stranded DNAs shown in Tables 1 and 2 can delete the inserted nucleotides from the GFP coding sequence (SEQ ID NO: 37) in which 8 nucleotides were inserted to encode loss-of-function GFP, and can replace the substituted nucleotide with the original nucleotide in the GFP coding sequence (SEQ ID NO:38) in which one nucleotide has been substituted to encode truncated GFP. Figure 1 shows DNA-70 (70EGFPdelnonPOD in the figure), 70-10L (70EGFPdelnonPOD-10L in the figure) and 70-10L-40c2L (70EGFPdelnonPOD-10L-40c2L in the figure) as a loss-of-function GFP coding sequence (upper panel) and truncated GFP coding sequence (lower panel). In the upper panel of Fig. 1, the 8 nucleotides in the middle of the genomic DNA antisense strand are the primary editing sites, and the 8 nucleotides in the middle of the genomic DNA sense strand are the secondary editing sites. In the lower panel of Fig. 1, one nucleotide in the middle of the genomic DNA antisense strand is the primary editing site, and one nucleotide in the middle of the genomic DNA sense strand is the secondary editing site.

### Example 1: Genome editing by LNA-introduced genome-editing polynucleotide (deletion)

### 1) Preparation of cells for assay

HEK293T cells were cultured in Dulbecco's Modified Eagle's Medium-high glucose (DMEM; Sigma, D5796) containing 10% (v/v) Fetal Bovine Serum (Sigma, F7524), 1% (v/v) Antibiotic-Antimycotic (Gibco, 15240-062), at 37°C under 5% CO2 conditions unless otherwise stated.

A cassette containing a loss-of-function GFP gene (SEQ ID NO: 37) was created by inserting 8 nucleotides within the GFP coding sequence and introduced into pCDH-CMV-MCS-EF1-RFP-T2A-Puro (System Biosciences, CD516B-2) to creat an expression vector pCDH-CMV-EGFP-nonPOD-1se-polyA-EF1-RFP+puro. This expression vector was transfected into HEK293T cells using Fugene HD (Promega). Transfected HEK293T cells were passaged at 4-6 day intervals in medium containing 0.25 or 0.5 µg/ml of puromycin to drug-select HEK293T cells in which the above cassette was introduced into the genome. The loss-of-function GFP expressed by the cells has a shifted reading frame due to the insertion of 8 nucleotides, resulting in an inactive form of the translated protein, which does not exhibit fluorescence.

### 2) Genome editing by LNA-introduced genome-editing polynucleotide

HEK293T cells expressing loss-of-function GFP prepared in step 1) were inoculated into 6-well plates (Corning, 3516) at 2.5×10⁵ cells/well and incubated in medium containing 10% (v/v) Fetal Bovine Serum (Sigma, F7524), 1% (v/v) Antibiotic-Antimycotic (Gibco, 15240-062) for 2 days to achieve 60-80% confluent cells. Then, 150 µl of DNA mixture (Opti-MEM (Gibco, 11058-021) + FuGENE HD Transfection Reagent (Promega)) containing 5 µg of one of the genome-editing polynucleotides listed in Tables 1 and 2 was added to each well, and incubated for another 1 day. After removal of the medium, cells were detached and collected using 0.025% Trypsin-EDTA (Gibco, 25300-062), seeded into 10 cm dishes, and cultured in medium containing 1% (v/v) Antibiotic-Antimycotic for 4 days. After removing the medium, cells were detached and collected using 0.025% Trypsin-EDTA.

### 3) Evaluation of genome editing efficiency by flow cytometry

Cells collected in step 2) were resuspended in medium containing 10% (v/v) Fetal Bovine Serum (Sigma, F7524) and 1% (v/v) Antibiotic-Antimycotic, and analyzed by flow cytometry (Attune NxT Flow Cytometer, Thermo Fisher Scientific) to count green fluorescent cells, and the percentage of green fluorescent cells to total cells was calculated as genome editing efficiency. The green fluorescent cells were those expressing normal GFP protein, meaning that the 8 nucleotides inserted in the nucleotide sequence encoding the loss-of-function type GFP were deleted by genome editing in those cells.

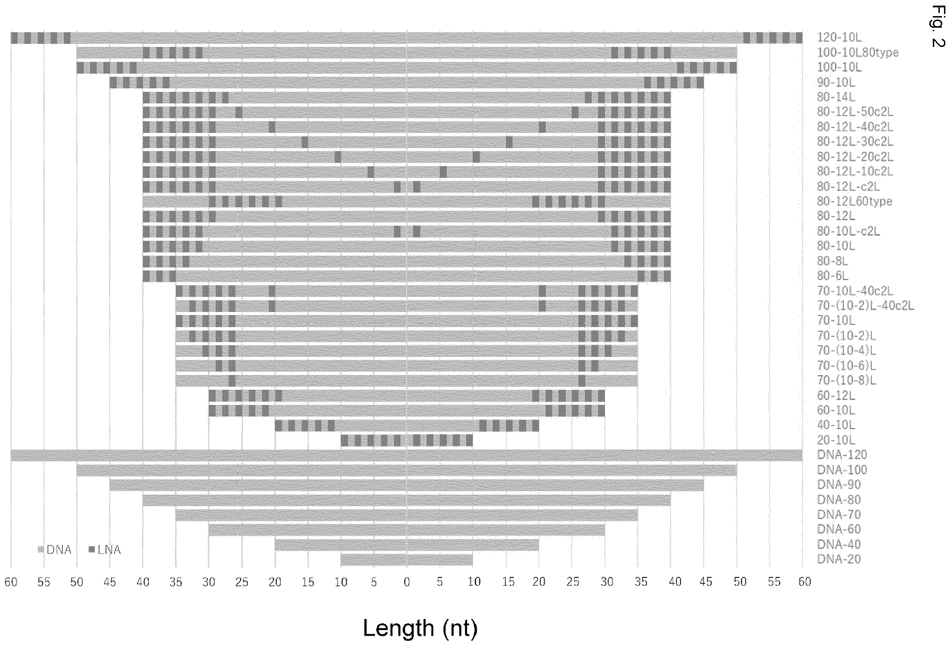

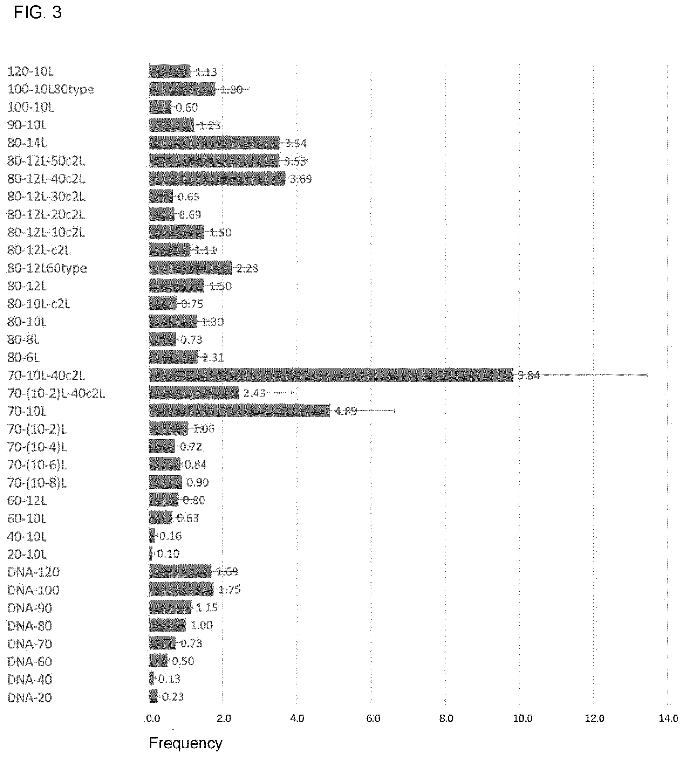

The positions of LNA introduction in each LNA-introduced genome-editing polynucleotide are shown in Fig. 2, and the efficiencies of genome editing are shown in Fig. 3. The length shown in Fig. 2 indicates the distance (number of nucleotides) from the deletion site. The frequencies shown in Figure 3 indicate the genome editing efficiency using each of the genome-editing polynucleotides, expressed as a relative value to the genome editing efficiency of 0.0005% using the genome-editing polynucleotide of 80 nucleotides in length without LNA (DNA-80 in the figure).

The genome-editing polynucleotides (80-14L, 80-12L-50c2L, 80-12L-40c2L, 70-10L-40c2L, 70-10L in the figure) having 3 or more LNAs at positions 30-38 from the 3' terminal of portion a) and 5' terminal of portion b), respectively, and having 1 or more LNAs at positions 20-29 from the 3' terminal of portion a) and 5' terminal of portion b), respectively, all showed improved genome editing efficiency compared to genome-editing polynucleotides of the same nucleotide length without LNA (DNA-80 or DNA-70 in the figure).

### Example 2: Genome editing by LNA-introduced genome-editing polynucleotide (substitution)

### 1) Preparation of cells for assay

A cassette containing a truncated GFP gene (SEQ ID NO:38) was created by substituting one nucleotide in the GFP coding sequence to form a termination codon in the middle of the GFP amino acid sequence, and introduced into pCDH-CMV-MCS-EF1-RFP-T2A-Puro (System Biosciences, CD516B-2) to create an expression vector (pCDH-CMV-EGFP80stop-EF1-RFP-T2A-puro). This expression vector was transfected into HEK293T cells using Fu GENE HD (Promega). Transfected HEK293T cells were passaged at 4-6 day intervals in medium containing 0.25 or 0.5 µg/ml of puromycin to drug-select HEK293T cells in which the above cassette was introduced into the genome. The truncated GFP expressed by the cells does not exhibit fluorescence.

### 2) Genome editing by LNA-introduced genome-editing polynucleotide and evaluation of genome editing efficiency

Using the genome-editing polynucleotides listed in Table 1, genome editing was performed in the same manner as in Example 1, 2) to 3), and the efficiency was evaluated. The genome editing efficiency was also evaluated by next-generation sequencing. Genomic DNA was extracted from cells collected in the same manner as in Example 1, 2) using the NucleoSpin Tissue Kit (Macherey-Nagel), and samples for sequencing were prepared using 2-step PCR referring to the TruSeq Adapter sequence (Illumina). Sequencing was performed using a next-generation sequencer (iSeq 100 Sequencing System, Illumina). The genome editing efficiency was calculated by determining the percentage of reads with the substituted nucleotide sequence among the reads containing sequences before and after the nucleotide substitution site.

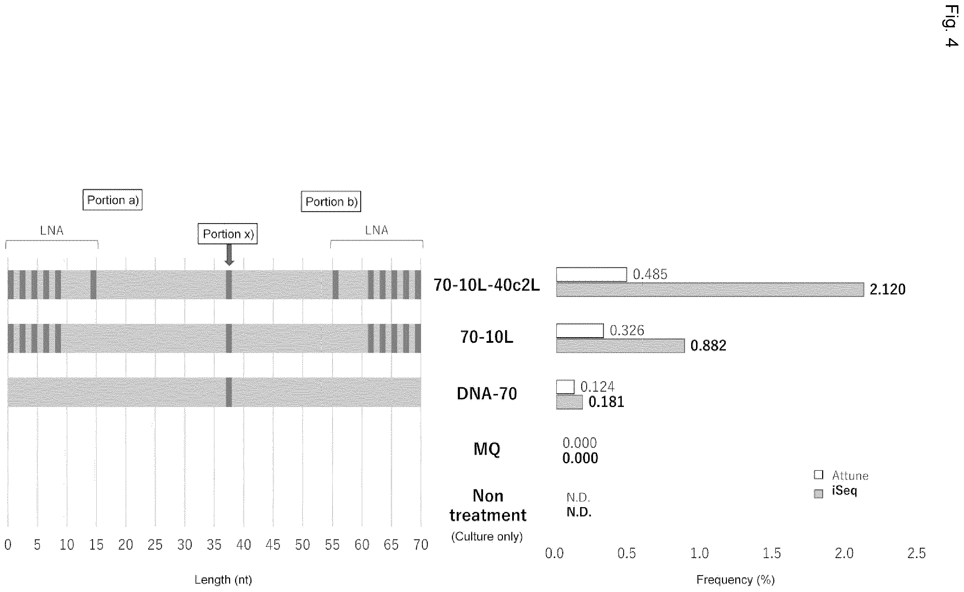

The positions of LNA introduction in the LNA-introduced genome-editing polynucleotides used are shown on the left side of Fig. 4, and the efficiencies of genome editing are shown on the right side of Fig. 4. The length shown on the left side of Fig. 4 indicates the distance (number of nucleotides) from the 5' terminal of the LNA-introduced genome-editing polynucleotide. The frequencies shown on the right side of Figure 4 indicate the genome editing efficiency using each of the genome-editing polynucleotides, where Attune means the result of evaluation by flow cytometry and iSeq means the result of evaluation by next-generation sequencing. MQ in Fig. 4 is ultrapure water.

The genome-editing polynucleotides 70-10L-40c2L and 70-10L, having 3 or more LNAs at positions 30-38 from the 3' terminal of portion a), and having 1 or more LNAs at positions 20-29 from the 3' terminal of portion a), showed improved genome editing efficiency compared to genome-editing polynucleotide of the same nucleotide length without LNA (DNA-70 in the figure).

### Example 3: Genome editing by combination of LNA-introduced genome-editing polynucleotide and promoting polynucleotide for genome editing

Single-stranded DNA (SEQ ID NO: 41) containing LNAs as part of the nucleotide sequence or single-stranded DNA (DNA-90, SEQ ID NO: 6) of the same nucleotide length without LNA was used as a genome-editing polynucleotide, and two additional single-stranded DNAs (SEQ ID NOs: 39 and 40) were used as promoting polynucleotides for genome-editing, to perform genome editing in the assay cells prepared in Example 2, 1) in the same manner as in Example 1, 2) to 4), and the efficiency of genome-editing was evaluated. A total of 15 µg of genome-editing polynucleotide and two types of promoting polynucleotide for genome editing, 5 µg each, were used.

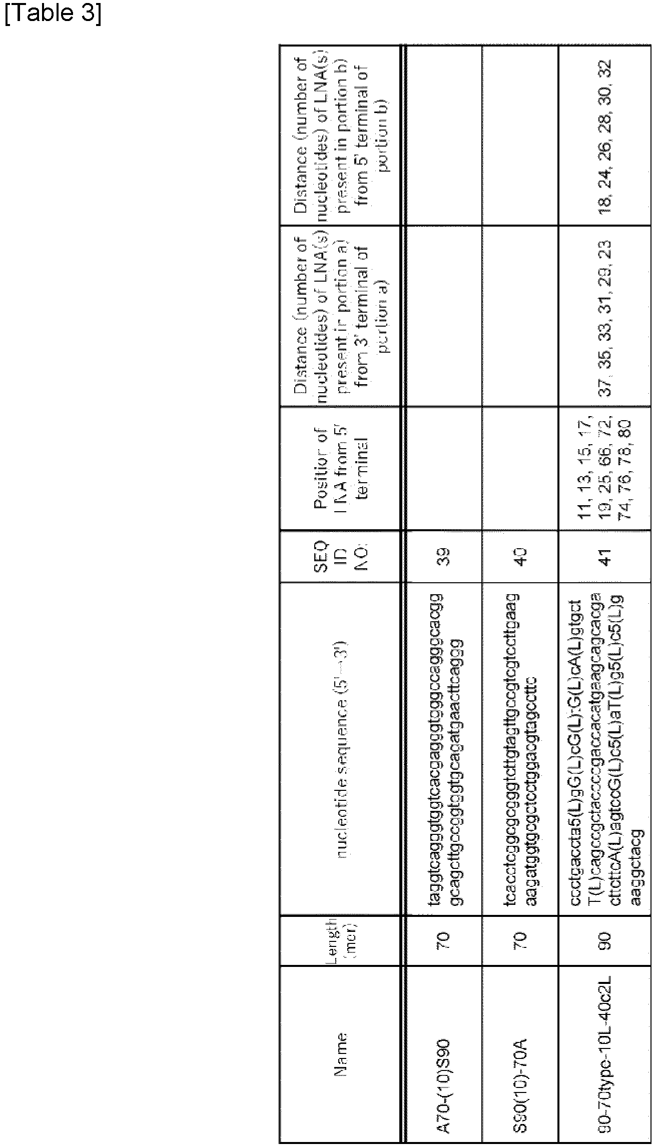

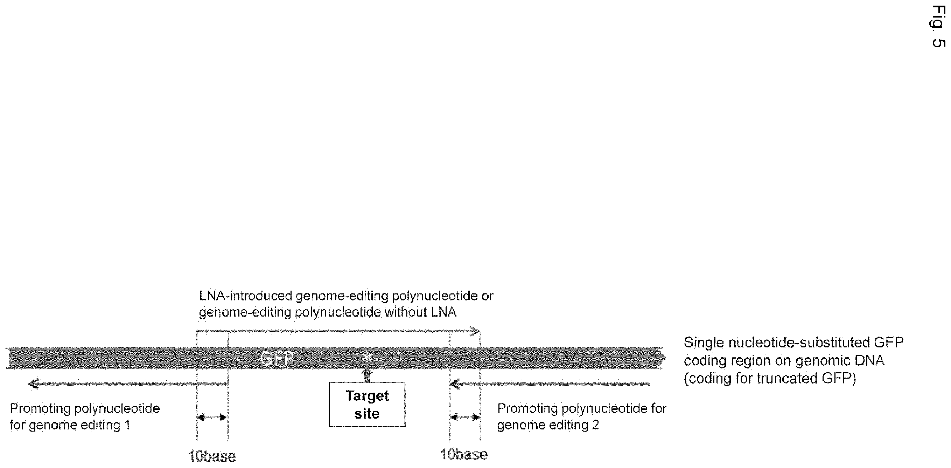

The two types of promoting polynucleotide for genome editing are designed to consist of a nucleotide sequence identical to a part of genomic DNA antisense strand, and to have 10 nucleotides at its 5' terminal part overlap with the DNA antisense strand region to which the 5' terminal part of the genome-editing polynucleotide is bound (promoting polynucleotide for genome editing 1), or to consist of a nucleotide sequence identical to a part of genomic DNA antisense strand, and to have 10 nucleotides at its 3' terminal part overlap with the DNA antisense strand region to which the 3' terminal part of the genome-editing polynucleotide is bound (promoting polynucleotide for genome editing 2). Figure 5 shows the positional relationship between the genomic DNA to be edited, the genome-editing polynucleotide, and the two types of promoting polynucleotides for genome editing.

Table 4 shows the results of the evaluation of genome editing efficiency by next-generation sequencing. A significant increase in genome editing efficiency was observed in genome editing performed by introducing the genome-editing polynucleotide (90-70type-10L-40c2L), having 3 or more LNAs at positions 30-38 from the 3' terminal of portion a) and 1 or more LNAs at positions 20-29 from the 3' terminal of portion a), and the two types of promoting polynucleotides for genome editing (A70-90S(10) and 90S(10)-70A) together, compared to genome editing performed by introducing the genome-editing polynucleotide (DNA-90) without LNA and the two types of promoting polynucleotides for genome editing (A70-90S(10) and 90S(10)-70A) together. This indicates that genome-editing efficiency is greatly improved by using a genome-editing polynucleotide containing LNAs, even when genome editing is performed with the addition of a promoting polynucleotide for genome editing.

**[Table 4]**

| Genome-editing polynucleotide | Promoting polynucleotide for genome editing 1 | Promoting polynucleotide for genome editing 2 | Genome editing efficiency (%) |
|---|---|---|---|
| - (Ultrapure water) | - | - | 0.008 |
| DNA-90 | - | - | 0.044 |
| DNA-90 | A70-90S(10) | 90S(10)-70A | 0.060 |
| 90-70type-10L-40c2L | - | - | 0.514 |
| 90-70type-10L-40c2L | A70-90S(10) | 90S(10)-70A | 0.868 |

## Claims

1. A single-stranded genome-editing polynucleotide capable of modifying a target site on a double-stranded genomic DNA and containing DNA high-affinity nucleotide analogs,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b),
at least one of portions a) and b) has a length of 32 to 100 nucleotides, and contains three or more DNA high-affinity nucleotide analogs within the range of the 30th to 38th nucleotides, one or more DNA high-affinity nucleotide analogs within the range of the 20th to 29th nucleotides, and no DNA high-affinity nucleotide analog within the ranges of the 1st to 2nd nucleotides or the 7th to 16th nucleotides, in the direction from the terminal adjacent to the other portion of the polynucleotide toward the non-adjacent terminal,
the other of the portions a) and b) has a length of 10 to 100 nucleotides, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

2. The genome-editing polynucleotide according to claim 1, wherein the DNA high-affinity nucleotide analogs are not adjacent to each other.

3. The genome-editing polynucleotide according to claim 1 or 2, wherein the DNA high-affinity nucleotide analogs are nucleotides in which the oxygen atom at the 2' position and the carbon atom at the 4' position of the ribose ring are cross-linked.

4. A kit for genome editing, containing:
the genome-editing polynucleotide according to any one of claims 1 to 3, or
a conjugate of the genome-editing polynucleotide with one or more other substances.

5. The kit for genome editing according to claim 4, further containing:
a promoting polynucleotide for genome editing,
an expression vector thereof, or
a conjugate of the promoting polynucleotide for genome editing or the expression vector thereof with one or more other substances,
wherein the promoting polynucleotide for genome editing is capable of hybridizing to a nucleic acid sequence that is complementary to a predetermined region on the genomic DNA strand on which the primary editing site is set,
the predetermined region overlaps at its 5' terminal part with region A or overlaps at its 3' terminal part with region B on the genomic DNA strand on which the primary editing site is set, in which a region on the genomic DNA strand at a position corresponding to portion a) of the genome-editing polynucleotide is defined as region A, and a region on the genomic DNA strand at a position corresponding to portion b) of the genome-editing polynucleotide is defined as region B,
the length of overlap with region A at the 5' terminal part of the predetermined region is the same as or shorter than region A, and the length of overlap with region B at the 3' terminal part of the predetermined region is the same as or shorter than region B, and
the promoting polynucleotide for genome editing has a length of 50 nucleotides or more.

6. The kit for genome editing according to claim 5, wherein the overlap has a length of 5 nucleotides or more.

7. The kit for genome editing according to claim 5 or 6, containing:
the promoting polynucleotide for genome editing in which the predetermined region is set to overlap at its 5' terminal part with region A,
an expression vector thereof, or
a conjugate of the promoting polynucleotide for genome editing or the expression vector thereof with one or more other substances, and
the promoting polynucleotide for genome editing in which the predetermined region is set to overlap at its 3' terminal part with region B,
an expression vector thereof, or
a conjugate of the promoting polynucleotide for genome editing or the expression vector thereof with one or more other substances.

8. A method for modifying a target site on a double-stranded genomic DNA of a cell excluding human gametes and fertilized eggs, or a non-human organism, comprising the step of treating the cell or the organism using the genome-editing polynucleotide according to any one of claims 1 to 3, or the kit for genome editing according to any one of claims 4 to 7, provided that when the cell is a human cell excluding human gametes and fertilized eggs, the step is performed in vitro or ex vivo.

9. A method for producing a cell excluding human gametes and fertilized eggs, or a non-human organism in which a target site on a double-stranded genomic DNA has been modified, comprising the step of treating the cell or the organism using the genome-editing polynucleotide according to any one of claims 1 to 3, or the kit for genome editing according to any one of claims 4 to 7, provided that when the cell is a human cell excluding human gametes and fertilized eggs, the step is performed in vitro or ex vivo.
